(19) Europäisches Patentamt
European Patent Office
Office européen des brevets

(11) Veröffentlichungsnummer: **0 052 295**
**B1**

(12) # EUROPÄISCHE PATENTSCHRIFT

(45) Veröffentlichungstag der Patentschrift: **08.05.85**

(51) Int. Cl.⁴: **C 07 D 307/20**, C 07 D 493/04 // (C07D493/04, 307:00, 307:00)

(21) Anmeldenummer: **81109422.6**

(22) Anmeldetag: **30.10.81**

(54) **Verfahren zur Herstellung von Polyolen mit mindestens einem Oxacyclopentanring.**

(30) Priorität: **05.11.80 DE 3041673**

(43) Veröffentlichungstag der Anmeldung: **26.05.82 Patentblatt 82/21**

(45) Bekanntmachung des Hinweises auf die Patenterteilung: **08.05.85 Patentblatt 85/19**

(84) Benannte Vertragsstaaten: **BE CH DE FR GB IT LI NL SE**

(56) Entgegenhaltungen: **CHEMICAL ABSTRACTS, Band 72, Nr. 15, 13. April 1970, Seite 442, Nr. 79370p, Columbus, Ohio, USA S. ROPUSZYNSKI et al: "Preparation of anhydroglucitols from d-glucitol in the presence of an ion-exchanger"**

(73) Patentinhaber: **CPC INTERNATIONAL INC., International Plaza P.O. Box 8000, Englewood Cliffs New Jersey 07632 (US)**

(72) Erfinder: **Feldmann, John, Dr., Dipl.-Chem., Grenzstrasse 151, D-4150 Krefeld (DE)**
Erfinder: **Koebernik, Hubert, Dr., Dipl.-Chem., Hohenzollernstrasse 76, D-4150 Krefeld (DE)**
Erfinder: **Woelk, Hans-Ulrich, Dr., Dipl.-Chem., Friedrich Kirstenstrasse 3, D-2000 Hamburg 65 (DE)**

(74) Vertreter: **Deufel, Paul, Dr. et al, Patentanwälte Müller-Boré, Deufel, Schön, Hertel Lewald, Otto Isartorplatz 6 Postfach 26 02 47, D-8000 München 26 (DE)**

**EP 0 052 295 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren der eingangs bezeichneten Art.

Die danach erhältlichen Anhydrozuckeralkohole der Formel I sind seit langem bekannte Verbindungen, die zum Teil unmittelbar, insbesondere für pharmazeutische Zwecke eingesetzt oder als Ausgangsmaterialien oder Zwischenprodukte für chemische Synthesen, beispielsweise von chiralen Feinchemikalien, Emulgatoren auf Esterbasis, Polyester- und Alkydharzen, sowie insbesondere von Polyurethanen, wobei sie als Starter und zur Vernetzung dienen, verwendet werden.

Zur Herstellung dieser Verbindungen wird bekanntlich bevorzugt eine säurekatalysierte, intramolekulare Wasserabspaltung an Zuckeralkoholen durchgeführt. Besonders eingehend untersucht und optimiert wurde diese Reaktion bezüglich des am besten zugänglichen Zuckeralkohols, des Sorbits.

Behandelt man D-Sorbit in Gegenwart einer Säure thermisch, so bildet sich zunächst ein Monoanhydrid, 1,4-Anhydro-D-sorbit, das unmittelbar zum 1,4;3,6-Dianhydro-D-sorbit weiterreagiert. Nebenprodukte dieser Reaktion können 2,5-Monoanhydrohexite sowie, in geringem Umfang, 3,6-Anhydrosorbit sein.

Üblicherweise wird diese Reaktion heute mit homogenen, also molekulardispers im Reaktionssystem verteilten Katalysatoren, wie Salzsäure oder Schwefelsäure duchgeführt. Die Verwendung dieser Säuren führt jedoch zu Nebenprodukten, insbesondere Estern der Katalysatorsäure, etwa durch Halogenierungen, und erfordert eine Neutralisation des Reaktionsproduktgemisches mit nachfolgender Entsalzung. Außerdem sind die dabei verwendeten Säuren stark korrosiv, teilweise zu leichtflüchtig und liefern Ausbeuten von maximal 66%, die nicht voll befriedigen können (Carbohydrate Chemistry, Vol. II, 191–198 (1963)).

Die ebenfalls bekannte Verwendung von organischen Lösungsmitteln zur Durchführung von Reaktionen dieses Typs unter azeotroper Entfernung des Reaktionswassers ergibt auch kaum nennenswerte Verbesserungen.

Einige der Nachteile der Verfahren mit im Reaktionsgemisch gelösten Säurekatalysatoren, wie die Korrosionsprobleme und die Bildung excessiver Mengen nur schwer abtrennbarer veresterter Nebenprodukte, lassen sich zwar in bekannter Weise durch die Verwendung heterogener Katalysatoren, nämlich (bestimmter) stark saurer Kationenaustauscherharze vermeiden, jedoch muß dies durch einige andere Nachteile, insbesondere noch schlechtere Ausbeuten von nur bis zu 35% und die Notwendigkeit, organische Lösungsmittel als Reaktionsmedium und Schleppmittel für die Entfernung von Wasser durch Azeotropdestillation zu verwenden, erkauft werden (vgl. Ropuszynski und Mit. in Przem. Chem. 1969, 48 (11), 665–8 und Weisleder und Mit. in Carbohydr. Res. 79, 133–141 (1980)).

Was die Notwendigkeit der Verwendung sorgfältig ausgewählter organischer Lösungsmittel(gemische) betrifft, wird in der letztgenannten Arbeit darauf hingewiesen, daß bei dem – zum Vergleich durchgeführten – Versuch 1,4;3,6-Dianhydro-D-sorbit aus Sorbit durch »trockene Destillation mit dem sauren Harz« herzustellen eine noch mehrfach geringere Ausbeute von nur 5% erzielt wurde.

Der Erfindung lag deshalb die Aufgabe zugrunde, ein Verfahren zur Herstellung von Polyolen der Formel I zu schaffen, das die Nachteile des Standes der Technik überwindet, d.h. insbesondere einerseits ohne die bei den mit homogenen Säurekatalysatoren auftretenden Probleme bezüglich Nebenproduktbildung und Korrosion zumindest ebenso hohe Ausbeuten liefert und andererseits den bekannten heterogenen katalysierten Verfahren bezüglich der erzielbaren Ausbeuten, sowie dadurch überlegen ist, daß keine kostspieligen organischen Lösungsmittel mitverwendet zu werden brauchen.

Diese Aufgabe wird erfindungsgemäß in der im Hauptanspruch gekennzeichneten Weise ausgehend von der überraschenden Erkenntnis gelöst, daß bei der Wasserabspaltung aus Zuckeralkoholen in Gegenwart heterogener Katalysatoren die Ausbeute in Abwesenheit organischer Lösungsmittel nicht nur nicht abfällt, sondern Ausbeuten erzielt werden, die weit, ja fallweise sogar um ein Mehrfaches über den im günstigsten Fall mit bekannten Verfahren dieses Typs erzielten Werten liegen, wenn man den im Kennzeichen des Hauptanspruchs angegebenen Auswahlregeln entsprechende Reaktionsbedingungen einhält.

Eine sinnfällige Erklärung für den erfindungsgemäß ausweislich der Beispiele erzielten Erfolg läßt sich auch im nachhinein nicht geben, da an sich zu erwarten wäre, daß

a) höhere Temperaturen und der durch Lösungsmittel bewirkte innigere Kontakt bzw. verbesserte Materialtransport zum und vom heterogenen Katalysator die Einstellung des Reaktionsgleichgewichts zumindest beschleunigen sollten und
b) die bei »trockener Destillation« erfolgende laufende Abtrennung beider Haupt-Reaktionsprodukte aus dem System die Anhydropolyolbildung begünstigen würde.

Die Erfindung, ihre Vorteile und ihre vorteilhafte Ausgestaltung werden nachfolgend vorwiegend am Beispiel des derzeit wirtschaftlich bedeutungsvollsten Zuckeralkohols, Sorbit, erläutert.

Als heterogene Katalysatoren kommen außer stark sauren Kationenaustauscherharzen im Prinzip alle an sich bekannten stark sauren festen Katalysatoren mit ausreichender Temperaturbeständigkeit und insbesondere »saure« Molekularsiebe, wie Zeolithe, und die vor allem in der Mineralölindustrie gebräuchlichen Crack- und/oder Hydrocrackkatalysatoren in Betracht, die für die Zwecke der Erfindung vor allem deswegen

von Interesse sind, weil sie auch für die Herstellung von Zuckeralkoholen durch katalytische Hydrierung der entsprechenden Zucker verbreitete Anwendung finden und somit den Weg für integrierte, ein- oder wenigstufige Verfahren zur Herstellung von Polyolen der Formel I aus Zuckern eröffnen.

Am besten haben sich bislang jedoch starksaure synthetische Kationenaustauscherharze und davon wiederum insbesondere mit Divinylbenzol vernetzte Polystyrolsulfonsäure-Kationenaustauscherharze bewährt. Dabei kommen sowohl sognenannte Gelharze, die nur schwach, also mit höchstens 4% Divinylbenzol vernetzt sein sollten, als auch makroporöse Harze in Betracht, die zweckmäßigerweise stark, d.h. mit mindestens 10% Divinylbenzol vernetzt sein sollten.

Der Katalysator wird beim Verfahren der Erfindung durch einfache Fest/Flüssig-Trennmethoden, z.B. Filtration vom Reaktionsproduktgemisch getrennt und kann erfahrungsgemäß ohne Aufbereitung mehrfach wiederverwendet werden.

Wegen der Oxidationsempfindlichkeit der Reaktionsteilnehmer und/oder -produkte und – fallweise – auch der Katalysatoren, wird das Verfahren der Erfindung vorzugsweise in einer Inertgas-, zweckmäßigerweise einer Stickstoffatmosphäre durchgeführt, wobei es in der Regel vorteilhaft ist, das Inertgas durch das Reaktionsgemisch perlen zu lassen, da dadurch u.a. eine gute Rührwirkung erzielt und zugleich die destillative Entfernung von Wasser aus dem Reaktionsgemisch gefördert wird.

Gemäß einer bevorzugten Ausführungsform der Erfindung wird die Umsetzung unter vermindertem Druck, insbesondere einem Druck im Bereich von 100 bis 50 000 Pa, durchgeführt. Dies empfiehlt sich insbesondere dann, wenn ein relativ hochschmelzendes Ausgangsmaterial eingesetzt und dessen Schmelzpunkt durch Zusatz von Fremdwasser unter die erfindungsgemäß einzuhaltende Höchsttemperatur von 160, vorzugsweise 155 und insbesondere 145°C gedrückt oder als Ausgangsmaterial eine wäßrige Zuckeralkohollösung verwendet und in situ eingeengt wird. Im letztgenannten Fall wird das Vakuum selbstverständlich zweckmäßigerweise erst dann angelegt und allmählich verstärkt, wenn der Wassergehalt auf einen Wert gesunken ist, bei dem das Reaktionsgemisch unter Normaldruck nicht mehr in dem erfindungsgemäß bevorzugten Reaktionstemperaturbereich von 100 bis 150 und insbesondere 110 bis 140°C siedet.

Als Ausgangsmaterial sind für die Zwecke der Erfindung vor allem Hexite und insbesondere Sorbit bevorzugt, da diese Zuckeralkohole leicht, preiswert und in praktisch unbegrenzten Mengen erhältlich sind und das Verfahren der Erfindung gerade hier hohe Ausbeuten, von mindestens 50, meist über 60 und im Falle von Sorbit sogar 85 bis 90% und darüber liefert. Ein weiteres bevorzugtes Ausgangsmaterial ist Erythrit, der in bekannter Weise aus Glykose gewonnen wird und ein in großen Mengen verfügbares Ausgangsmaterial darstellt und ein Anhydropolyol mit interessanten anwendungstechnischen Eigenschaften liefert. Ferner sei darauf hingewiesen, daß erfindungsgemäß auch Zuckeralkoholgemische, z.B. Sorbit/Mannit-Gemische, wie es beispielhalber bei der katalytischen Hydrierung von Glukose/Fruktose-Gemischen anfällt, eingesetzt werden können; ein diesbezüglich interessantes Ausgangsmaterial stellen $C_4-C_6$-Polyolgemische dar, die man durch Hydrieren von Hemicellulosehydraten erhält. Solche Hydrolysate werden derzeit aus Nebenprodukten, wie z.B. Haferschalen, Maisschalen etc. in großen Mengen industriell hergestellt, und zwar im wesentlichen zur Gewinnung von Xylose bzw. Xylit. Die dabei zwangsläufig anfallenden Tetrosen, anderen Pentosen und Hexosen bzw. deren Hydrierungsprodukte stellen vorläufig immer noch kaum verwertbare Abfallprodukte dar, die nach dem erfindungsgemäßen Verfahren in ein wertvolles Zwischenprodukt, insbesondere für die Kunststoffindustrie, umgewandelt werden können.

Das Verfahren der Erfindung kann nicht nur absatzweise, sondern auch kontinuierlich durchgeführt werden, wobei dann Rührkessel-und/oder Wirbelschichtreaktoren besonders zweckmäßig sind und in der Regel eine zwei- oder mehrstufige Arbeitsweise – vor allem wegen der dabei einfacheren Anpassung der Reaktionsbedingungen an die Zusammensetzung des Reaktionsgemisches – vorzuziehen ist.

Die erfindungsgemäß erhaltenen Reaktionsproduktgemische können – vor allem für technische Zwecke – wie etwa als Polyolkomponente in Kunstharzsynthesen – häufig direkt, d.h. ohne weitere Aufarbeitung und/oder Reinigung eingesetzt werden. Soweit ein höherer Reinheitsgrad im Hinblick auf die bestimmungsgemäße Verwendung erforderlich oder zumindest erwünscht erscheint, können die rohen Reaktionsproduktgemische nach beliebigen an sich bekannten Methoden, z.B. durch fraktionierte Kristallisation, Destillation oder Säulenchromatographie, aufgearbeitet und/oder – z.B. durch Behandlung mit Aktivkohle und/oder Ionenaustauscherharzen – gereinigt werden, wobei sich die Gesamtausbeute dadurch erhöhen läßt, daß bei einer Abtrennung anfallende Fraktionen mit hohem Gehalt nicht umgesetzten Ausgangsmaterials und/oder – im Falle von Hexiten – Monoanhydropolyolen im Kreislauf in die Reaktion zurückführt.

Die Beispiele erläutern die Erfindung.

## Beispiel 1

Ein handelsüblicher Rührkessel wurde mit 25 kg einer 70%igen Sorbitlösung und 1 kg eines stark sauren Ionenaustauschers in der $H^+$-Form (makroporöses Polystyrolsulfonsäureharz, 14% DVB-vernetzt) beschickt. Das Gemisch wurde zur Trockne eingedampft und dann zwei Stunden bei einem Druck von 3000 Pa und 140°C weitergerührt.

Die erhaltene Schmelze wurde heiß vom Ionen-

austauscher abfiltriert. Dabei wurden 13 900 g Rohprodukt erhalten, das 91 Gew.-% Dianhydrosorbit enthielt.

Beispiel 2

In einem 1 Ltr. Dreihalsglaskolben wurden 250 g Sorbit und 20 g des in Beispiel 1 beschriebenen Ionenaustauschers vorgelegt und analog Beispiel 1 weiterbehandelt. Nach 3 Stunden Reaktionsdauer wurde wie beschrieben filtriert. Dabei wurden 190 g Rohprodukt erhalten, das 87 Gew.-% Dianhydrosorbit enthielt.

Beispiel 3

Beispiel 2 wurde wiederholt, wobei jedoch abweichend davon ein mit 20% Divinylbenzol vernetztes makroporöses Polystyrolsulfonsäureharz als Katalysator benutzt und eine Reaktionsdauer von nur 2 Stunden angewandt wurde. Es wurden 191 g Rohprodukt erhalten, das 89 Gew.-% Dianhydrosorbit enthielt.

Beispiel 4

Beispiel 3 wurde wiederholt, wobei abweichend davon als Katalysator ein mit 2% DVB vernetztes, stark saures Polystyrolsäure-Gel-Ionenaustauscherharz (in der H-Form) verwendet wurde. Es wurden 191 g Rohprodukt erhalten, das 87 Gew.-% Dianhydrosorbit enthielt.

Beispiel 5

Beispiel 4 wurde wiederholt, wobei abweichend davon als Katalysator ein mit 10% DVB (stark) vernetztes Gelharz eingesetzt und die Reaktionszeit auf 5 Stunden verlängert wurde. Es wurden 190 g Rohprodukt erhalten, das 86 Gew.-% Dianhydrosorbit enthielt.

Beispiel 6

In einem 1 Ltr. Freihalskolben wurden 200 g Mannit, 50 ml Wasser und 30 g des in Beispiel 3 beschriebenen Ionenaustauschers vorgelegt und 2 Stunden bei 140°C gerührt, worauf unter allmählicher Verringerung des Drucks auf 3000 Pa bei konstanter Temperatur Wasser verdampft und das Reaktionsgemisch dann unter konstanten Bedingungen weitere 5 Stunden gerührt und danach der Katalysator heiß abfiltriert wurde. Dabei wurden 151 g Rohprodukt erhalten, das 76 Gew.-% Dianhydromannit enthielt.

Beispiel 7

In einem 1 Ltr. Dreihalskolben wurden 250 g einer Mischung aus gleichen Gewichtsteilen Sorbit und Mannit und 30 g des in Beispiel 3 beschriebenen Katalysators vorgelegt, 4 Stunden bei 3000 Pa und 145°C gerührt, worauf der Katalysator heiß abfiltriert wurde. Dabei wurden 192 g Rohprodukt erhalten, das 46 Gew.-% Dianhydrosorbit und 40 Gew.-% Dianhydromannit enthielt.

Beispiel 8

Analog Beispiel 3 wurden 200 g meso-Erythrit 4 Stunden bei 0,03 bar und 140°C gerührt, wobei während der Umsetzung Stickstoff durch das Reaktionsgemisch perlen gelassen wurde. Es wurden 175 g Rohprodukt erhalten, das 86 Gew.-% Anhydroerythrit enthielt.

Beispiel 9

Beispiel 2 wurde wiederholt, wobei jedoch die Reaktion erst nach 5 Stunden beendet wurde. Das Reaktionsgemisch wurde wie üblich filtriert, wobei 189 g Rohprodukt erhalten wurden, das 94 Gew.-% Dianhydrosorbit enthielt.

Beispiel 10

In einem 1 Ltr. Dreihalskolben wurden 250 g eines katalytisch hydrierten wasserfreien Hemicellulosehydrolysates aus Maisschalen und 20 g eines stark sauren Ionenaustauschers in der $H^+$-Form (Polystyrolsulfonsäure — Gelharz, 2 % DVB-vernetzt) vorgelegt und 2 Stunden bei 130°C und einem Druck von 3000 Pa gerührt.

Die erhaltene Schmelze wurde heiß vom Ionenaustauscher filtriert, mit Wasser aufgenommen und mit Aktivkohle behandelt. Nach Filtrieren und Einengen wurden 180 g Produkt erhalten, das eine Hydroxylzahl von 985 im Vergleich zu 1790 beim Ausgangsmaterial aufwies.

Eine vergleichende HPLC-Studie zeigte, daß die im Rohstoff vorhandenen Polyole (ca. 75 % Pentite, davon über 55 % Xylit, sowie Sorbit, Galaktit) sich zu über 98 % in Anhydroderivate umgesetzt hatten.

**Patentansprüche**

1. Verfahren zur Herstellung von Polyolen mit mindestens einem Oxacyclopentanring der allgemeinen Formel

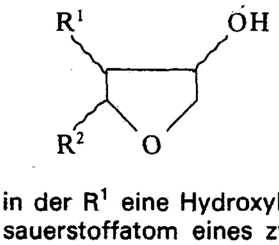

(I)

in der $R^1$ eine Hydroxylgruppe oder das Äthersauerstoffatom eines zweiten Oxacyclopentan-

rings und $R^2$ ein Wasserstoffatom, eine Hydroxymethylgruppe oder ein zweiwertiger Rest der Formel

$$-CH_2-CHOH-$$

ist, wobei im letztgenannten Fall $R^1$ ein Äthersauerstoffatom ist und $R^1$ und $R^2$ zusammen mit den Kohlenstoffatomen, an die sie gebunden sind, einen zweiten Oxacyclopentanring bilden, durch Wasserabspaltung aus Zuckeralkoholen mit 4 bis 6 Kohlenstoffatomen bei erhöhter Temperatur in Gegenwart eines stark sauren, heterogenen Katalysators und in Abwesenheit organischer Lösungsmittel, wobei im Verlauf der Umsetzung etwaiges mit den Ausgangsmaterialien eingeschlepptes Fremdwasser praktisch vollständig und sich im Zuge der Umsetzung bildendes Reaktionswasser zumindest teilweise destillativ aus dem Reaktionsgemisch entfernt wird, dadurch gekennzeichnet, daß man die Umsetzung in einem schmelzflüssigen Reaktionssystem – vorzugsweise unter vermindertem Druck – bei Temperaturen von höchstens 160°C durchführt, mit der Maßgabe, daß die Temperatur in Abhängigkeit vom Druck so gewählt wird, daß vor Beendigung der Umsetzung bzw. Abtrennung des Katalysators praktisch keine destillative Abtrennung von Polyol-Reaktionsprodukt(en) aus dem Reaktionsgemisch erfolgt.

2. Verfahren nach Anspruch 1, dadurch gekennzeichnet, daß man als Katalysator ein stark saures Kationenaustauscherharz, stark saures anorganisches Molekularsieb und/oder einen sauren Crack- und/oder Hydrocrackkatalysator verwendet.

3. Verfahren nach Anspruch 2, dadurch gekennzeichnet, daß man als Katalysator ein mit Divinylbenzol vernetztes Polystyrolsulfonsäure-Kationenaustauscherharz in der H-Form verwendet.

4. Verfahren nach einem der Ansprüche 1 bis 3, dadurch gekennzeichnet, daß man die Umsetzung in einer Inertgasatmosphäre durchführt.

5. Verfahren nach Anspruch 4, dadurch gekennzeichnet, daß man das Inertgas durch das Reaktionsgemisch perlen läßt.

6. Verfahren nach einem der Ansprüche 1 bis 5, dadurch gekennzeichnet, daß man die Umsetzung unter vermindertem Druck im Bereich von 100 bis 50 000 Pa, vorzugsweise 1000 bis 20 000 Pa durchführt.

7. Verfahren nach einem der Ansprüche 1 bis 6, dadurch gekennzeichnet, daß man die Reaktionstemperatur höchstens auf 155 und vorzugsweise nicht mehr als 145°C ansteigen läßt.

8. Verfahren nach einem der Ansprüche 1 bis 7, dadurch gekennzeichnet, daß man bis zu 50 Gew.-% Fremdwasser enthaltende Lösung bzw. Schmelze des Ausgangsmaterials einsetzt.

9. Verfahren nach einem der Ansprüche 1 bis 8, dadurch gekennzeichnet, daß man als Ausgangsmaterial wenigstens ein Hexit, insbesondere Sorbit, oder ein $C_4-C_6$-Polyolgemisch, insbesondere ein hydriertes Hemicellulosehydrolysat, verwendet.

10. Verfahren nach Anspruch 9, dadurch gekennzeichnet, daß die Reaktion so lange fortgeführt wird, bis mindestens 50, vorzugsweise mindestens 65 % des Ausgangsmaterials zu den entsprechenden Anhydrotetriten, -pentiten und/oder Dianhydrohexiten umgesetzt wird.

11. Verfahren nach einem der Ansprüche 1 bis 10, dadurch gekennzeichnet, daß die Umsetzung kontinuierlich und vorzugsweise zwei- oder mehrstufig durchgeführt wird.

12. Verfahren nach Anspruch 11, dadurch gekennzeichnet, daß die Umsetzung in mindestens einem Rührkessel- oder Wirbelbettreaktor durchgeführt wird.

## Claims

1. A process for preparing polyoles having at least one oxacyclopentane ring of the generic formula

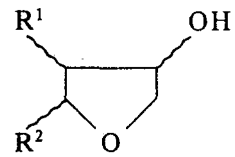

(I)

wherein $R^1$ is a hydroxyl group or the ether oxygen atom of a second oxacyclopentane ring, and $R^2$ being a hydrogen atom, a hydroxymethyl group or a bivalent redical of the formula

$$-CH_2-CHOH-$$

whereby in the latter case $R^1$ is a ether oxygen atom and $R^1$ and $R^2$ together with the carbon atoms with which they are attached form a second oxacyclopentane ring, by cleavage of water from sugar alcohols having 4 to 6 carbon atoms at elevated temperature in presence of a strongly acidic heterogeneous catalyst and in absence of organic solvents, outside water being introduced with the starting materials and during the reaction formed reaction water being at least partly destilled from the reaction mixture during the reaction, characterized in that the reaction is carried out in a melt liquid reaction system – preferably under reduced pressure – at temperatures of no more than 160°C, with the proviso that the temperature is selected in dependency of the pressure in such a way that prior to the completion of the reaction and the separation of the catalyst, respectively, essentially no destillativ preparation of polyole reaction product(s) from the reaction mixture takes place.

2. A process according to claim 1, characterized in that as catalyst a strongly acidic cation exchange resin, a strongly acidic inorganic molecular sieve and/or an acidic cracking and/or hydrochracking catalyst is used.

3. A process according to claim 2, characterized in that as catalyst a polystyrene sulfonic acid-cation exchange resin in the H-form crosslinkable with divinyl benzene is used.

4. A process according to one of the claims 1 to 3, characterized in that the reaction is carried out in an inert gas atmosphere.

5. A process according to claim 4, characterized in that the inert gas is bubbled through the reaction mixture.

6. A process according to one of the claims 1 to 5, characterized in that the reaction is carried out under reduced pressure in the range of 100 to 50000 Pa, preferably 1000 to 20000 Pa.

7. A process according to one of the claims 1 to 6, characterized in that the reaction temperature is allowed to increase to no more than 155 and preferably to no more than 145°C.

8. A process according to one of the claims 1 to 7, characterized in that a up to 50% by weight outside water containing solution and melt respectively, of the starting material is used.

9. A process according to one of the claims 1 to 8, characterized in that as starting material at least one hexite, especially sorbite, or a $C_4$–$C_6$-polyole mixture, especially a hydrogenated hemicellulose hydrolisate is used.

10. A process according to claim 9, characterized in that the reaction is carried out until at least 50 and preferably at least 65% of the starting material has been reacted to the corresponding unhydrotetrites, -pentites and/or dianhydrohexites.

11. A process according to one of the claims 1 to 10, characterized in that the reaction is carried out contineously and preferably in two or more stages.

12. A process according to claim 11, characterized in that the reaction is carried out in at least one stirred vessel or fluidized bed reactor.

**Revendications**

1. Procédé de production de polyols ayant au moins un cycle d'oxacyclopentane de formule générale

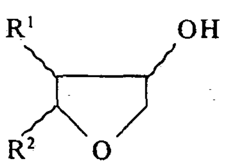

(I)

dans laquelle $R^1$ est un groupe hydroxyle ou l'atome d'oxygène d'éther d'un second cycle d'oxacyclopentane et $R^2$ est un atome d'hydrogène, un groupe hydroxyméthyle ou un reste divalent de formule

$$—CH_2—CHOH—$$

et dans le dernier cas, $R^1$ représente un atome d'oxygène d'éther et $R^1$ et $R^2$ forment conjointement avec les atomes de carbone auxquels ils sont liés un second cycle d'oxacyclopentane, par élimination d'eau d'alcool-sucres ayant 4 à 6 atomes de carbone à température élevée en présence d'un catalyseur hétérogène fortement acide et en l'absence de solvants organiques, et au cours de la réaction, l'eau étrangère éventuelle entraînée avec les matières de départ et l'eau de réaction qui se forme pendant la réaction étant éliminées du mélange réactionnel par distillation, la première à peu près totalement et la seconde au moins partiellement, caractérisé en ce qu'on conduit la réaction dans un système réactionnel liquide à l'état fondu — de préférence sous pression réduite — à des températures au maximum égales à 160°C, sous réserve que la temperature soit choisie en fonction de la pression de manière qu'avant la fin de la réaction et dè la séparation du catalyseur, il n'y ait pratiquement pas de séparation par distillation de polyol(s) obtenu(s) comme produit(s) réactionnel(s) du mélange réactionnel.

2. Procédé suivant la revendication 1, caractérisé en ce qu'on utilise comme catalyseur une résine d'échange cationique fortement acide, un tamis moléculaire inorganique fortement acide et/ou un catalyseur de craquage et/ou d'hydrocraquage acide.

3. Procédé suivant la revendication 2, caractérisé en ce qu'on utilise comme catalyseur une résine d'échange cationique du type d'un acide polystyrène-sulfonique réticulé au divinylbenzène, sous la forme H.

4. Procédé suivant l'une des revendications 1 à 3, caractérisé en ce qu'on conduit la réaction dans une atmosphère de gaz inerte.

5. Procédé suivant la revendication 4, caractérisé en ce qu'on fait passer le gaz inerte en bulles très fines dans le mélange réactionnel.

6. Procédé suivant l'une des revendications 1 à 5, caractérisé en ce qu'on conduit la réaction sous pression réduite dans l'intervalle de 100 à 50000 Pa, de préférence de 1000 à 20000 Pa.

7. Procédé suivant l'une des revendications 1 à 6, caractérisé en ce qu'on fait monter la température de réaction au maximum à 155°C et de préférence à une valeur ne dépassant pas 145°C.

8. Procédé suivant l'une des revendications 1 à 7, caractérisé en ce qu'on utilise une solution ou une masse fondue de la matière de départ contenant jusqu'à 50% en poids d'eau étrangère.

9. Procédé suivant l'une des revendications 1 à 8, caractérisé en ce qu'on utilise comme matière de départ au moins un hexitol, notamment du sorbitol, ou un mélange de polyols en $C_4$ à $C_6$, notamment un hydrolysat hydrogéné d'hémicellulose.

10. Procédé suivant la revendication 9, caractérisé en ce qu'on poursuit la réaction jusqu'à ce qu'au moins 50 et de préférence au moins 65% de la matière de départ aient été transformés en les anhydrotétrols, anhydropentols et/ou dianhydrohexitols corresdants en proportion d'au moins 50%, de préférence d'au moins 65% de la matière de départ.

11. Procédé suivant l'une des revendications 1 à 10, caractérisé en ce qu'on conduit la réaction en continu ou de préférence en deux ou plus de deux étapes.

12. Procédé suivant la revendication 11, caractérisé en ce qu'on conduit la réaction dans au moins un réacteur à récipient d'agitation ou à lit tourbillonnaire.